# EUROPÄISCHE PATENTANMELDUNG

(11) **EP 4 563 095 A1**
(43) Veröffentlichungstag der Anmeldung: **04.06.2025**
(21) Anmeldenummer: 24213190.2
(22) Anmeldetag: 15.11.2024
(51) Int. Cl.: A61B 17/00

(54) **WECHSELVORRICHTUNG FÜR ZUMINDEST ZWEI CHIRURGISCHE WECHSELINSTRUMENTE, CHIRURGISCHES ROBOTERSYSTEM UND VERFAHREN ZUM WECHSELN VON CHIRURGISCHEN WECHSELINSTRUMENTEN**

(30) Priorität: 28.11.2023 DE 102023133157
(71) Anmelder: Karl Storz SE & Co. KG, 78532 Tuttlingen (DE)
(72) Erfinder: STEFAN, Jochen, 78532 Tuttlingen (DE)

(57) **Zusammenfassung**

Die vorliegende Erfindung stellt eine Wechselvorrichtung (10) für chirurgische Wechselinstrumente (1) bereit. Von einem Instrumentenmagazin (14) für die Wechselinstrumente (1) in Passivstellung erstreckt sich eine Linearführung (12), um jeweils ein Wechselinstrument (1) auf einem Schlitten (13) von einer Wechselstellung in eine Aktivstellung zu überführen. Das Wechselinstrument (1) hat eine Handhabe (2) und einen Schaft (3), der in der Wechselstellung und der Aktivstellung parallel zu der Linearführung (12) ist. Das Instrumentenmagazin (14) weist eine Schienenführung (15, 16) mit einem Führungsprofil auf, das eine Kreisbahn definiert. Der Schlitten (13) hat zumindest einen Schienenabschnitt (17, 18, 19), dessen Profil dem Führungsprofil der Schienenführung (15, 16) entspricht und der in der Wechselstellung die Schienenführung (15, 16) des Instrumentenmagazins (14) entlang der Kreisbahn ergänzt. Jedes Wechselinstrument (1) weist an der Handhabe (2) zumindest ein Eingriffselement (22, 23) mit einem Eingriffsprofil auf, das komplementär zu dem Führungsprofil der Schienenführung (15, 16) und des Schienenabschnitts (17, 18, 19) ausgebildet ist, sodass jedes Wechselinstrument (1) in der Passivstellung auf der Kreisbahn des Instrumentenmagazins (14) bewegbar und in der Wechselstellung auf dem Schlitten (13) positionierbar ist. Ferner werden ein chirurgisches Robotersystem und ein Verfahren zum Wechseln von chirurgischen Wechselinstrumenten (1) offenbart.

## Beschreibung

Die Erfindung betrifft eine Wechselvorrichtung für zumindest zwei chirurgische Wechselinstrumente, ein chirurgisches Robotersystem mit einer solchen Wechselvorrichtung und Verfahren zum Wechseln von chirurgischen Wechselinstrumenten unter Verwendung der Wechselvorrichtung.

Aus dem Stand der Technik sind Master-Slave-Robotersysteme für die Chirurgie bekannt, die direkt am Patienten arbeiten und von einem unsterilen Operateur an einer Eingabekonsole gesteuert werden. Mittels der Eingabekonsole können Werkzeuge an chirurgischen Instrumenten bedient werden, die meist durch Trokare in den Körper des Patienten hineinragen. Geführt und angetrieben werden diese Instrumente durch gelenkige Roboterarme, die in einem komplexen Zusammenspiel aller Freiheitsgrade (von Roboterarm und Instrument), die Bewegungseingaben des Operateurs (an der Konsole) in Bewegungen der Instrumentenspitze umsetzen. Werden für eine Operation an einem Roboterarm unterschiedliche Instrumente benötigt, muss das Instrument bei den bekannten Systemen von einer sterilen Person in direkter Patientenumgebung von Hand gewechselt werden. Hierzu muss manuell die Schnittstelle des zu entnehmenden und idealerweise bereit aus dem Trokar zurückgezogenen Instrumentes zum Roboter gelöst werden und das Instrument entnommen werden. Anschließend wird das neue Instrument in umgekehrter Reihenfolge wieder eingeführt und mit dem Roboterarm verbunden.

Es ist somit nicht möglich die Operation nach Einrichtung des Systems vollständig von der Konsole aus durchzuführen. Es ist entweder ein Pendeln des Operateurs zwischen Konsole und steriler Patientenumgebung oder eine helfende Person in der Nähe des Patienten notwendig.

Aus DE 10 2009 025 013 B4 ist ein System bekannt, dass einen automatisierten Instrumentenwechsel ermöglicht, wobei zwei medizinische Instrumente in einem Instrumentenspeicher mit Speicherkammern vorliegen, die jeweils einem Arbeitskanal zugeführt werden können. Der Arbeitskanal kann jeweils das Instrument aufnehmen, das verwendet werden soll, dazu wird der Instrumentenspeicher wie ein Revolver gedreht, bis die Speicherkammer mit dem zu benutzenden Instrument mit dem Arbeitskanal axial ausgerichtet ist. Allerdings benötigt dieser Instrumentenspeicher eine komplexe Hydraulik, um die Instrumente vorzuschieben und Schaftinstrumente können mit diesem System nicht verwendet werden.

Ausgehend von diesem Stand der Technik ist es Aufgabe der vorliegenden Erfindung, eine verbesserte Wechselvorrichtung bereitzustellen, die es ermöglicht Schaftinstrumente zur Verwendung in einem chirurgischen Robotersystem einfach und automatisiert zu wechseln.

Diese Aufgabe wird durch eine Wechselvorrichtung mit den Merkmalen des Anspruchs 1 gelöst.

Die weitere Aufgabe ein chirurgisches Robotersystem bereitzustellen, mit dessen Wechselvorrichtung Schaftinstrumente einfach und automatisch gewechselt werden können, wird durch das Robotersystem mit den Merkmalen des unabhängigen Anspruchs 13 gelöst.

Die weitere Aufgabe, Schaftinstrumente zur Verwendung in einem chirurgischen Robotersystem in einfacher Weise und automatisch zu wechseln, wird durch das Verfahren mit den Merkmalen des unabhängigen Anspruchs 14 gelöst.

Weiterbildungen bzw. bevorzugte Ausführungsformen der Wechselvorrichtung, des chirurgischen Robotersystems oder des Verfahrens sind in den Unteransprüchen ausgeführt.

Gemäß einer ersten Ausführungsform ist die Wechselvorrichtung für zumindest zwei chirurgische Wechselinstrumente ausgebildet, die in der Wechselvorrichtung wahlweise in einer Aktivstellung, einer Wechselstellung und einer Passivstellung positionierbar sind. Dabei weist die Wechselvorrichtung ein Instrumentenmagazin zur Aufnahme der zumindest zwei Wechselinstrumente in der Passivstellung und eine Linearführung mit einem proximalen Ende, an dem das Instrumentenmagazin angeordnet ist, und einem distalen Ende, an dem das Wechselinstrument in der Aktivstellung angeordnet ist, auf. Die Wechselvorrichtung weist noch eine Antriebseinheit, die dazu ausgebildet ist, wahlweise jedes der Wechselinstrumente an dem proximalen Ende der Linearführung in einer Wechselstellung anzuordnen, und eine Überführungseinrichtung zum Überführen des Wechselinstrumente entlang der Linearführung zwischen der Wechselstellung am proximalen Ende in die Aktivstellung am distalen Ende auf.

Erfindungsgemäß weist jedes Wechselinstrument eine Handhabe, einen Schaft und ein Werkzeug auf, das an einem distalen Ende des Schafts angeordnet ist und mit der Handhabe operativ gekoppelt ist, die an einem proximalen Ende des Schafts angeordnet ist, der eine Instrumentenachse A definiert, die in der Wechselstellung und der Aktivstellung parallel zu einer Längsachse L der Wechselvorrichtung ist, wobei die Längsachse L durch die Linearführung definiert wird.

Ferner hat das Instrumentenmagazin eine Schienenführung mit einem Führungsprofil, das zumindest eine Kreisbahn definiert, die sich zu dem proximalen Ende der Linearführung erstreckt.

Die Überführungseinrichtung weist einen entlang der Linearführung bewegbaren Schlitten mit zumindest einem Schienenabschnitt auf, dessen Profil dem Führungsprofil der Schienenführung entspricht und der in der Wechselstellung die Schienenführung des Instrumentenmagazins entlang der zumindest einen Kreisbahn ergänzt.

Dabei hat jedes Wechselinstrument an der Handhabe zumindest ein Eingriffselement mit einem Eingriffsprofil, das komplementär zu dem Führungsprofil der Schienenführung und des Schienenabschnitts ausgebildet ist, sodass jedes Wechselinstrument in der Passivstellung auf der Kreisbahn des Instrumentenmagazins bewegt und in der Wechselstellung auf dem Schlitten positioniert werden kann.

"Wechselinstrument" bedeutet hierin ein chirurgisches Instrument mit einem Schaft, das in die Wechselvorrichtung eingesetzt werden kann und mittels der Wechselvorrichtung gewechselt werden kann.

Die "Handhabe" des Wechselinstruments umfasst alle Komponenten zur Ansteuerung und Bewegung des Werkzeugs am distalen Ende des Schafts des Instruments. Sie entspricht im Prinzip einer Getriebeeinheit eines chirurgischen Instruments.

Die "zumindest eine Kreisbahn" bezieht sich auf weiter unten näher beschriebene einzelne Strukturen, die Profile, Profilstrukturen oder ähnliches sein können und die in verschiedenen Radien parallel zueinander liegen können, um so eine Schienenführung zu bilden.

"Passivstellung", "Wechselstellung" und "Aktivstellung" bezeichnen die verschiedenen Positionen, die eines der zumindest zwei Wechselinstrumente in der Wechselvorrichtung einnehmen kann. Im Instrumentenmagazin liegen die zumindest zwei Wechselinstrumente zunächst in einer Passivstellung vor. Auf dem an dem proximalen Ende der Linearführung befindlichen Schlitten aufliegend nimmt das dort liegende Wechselinstrument eine Wechselstellung ein. Vorgeschoben an das distale Ende der Linearführung, eingeführt in den Trokar bzw. eine Trokarvorrichtung ist das auf dem Schlitten aufliegende Wechselinstrument in der Aktivstellung. Da sich die Wechselstellung und Aktivstellung des Wechselinstruments auf die Position des Schlittens in der Linearführung beziehen, werden die Begriffe Wechselstellung und Aktivstellung auch in Bezug auf den Schlitten in korrespondierender Weise verwendet, um dessen Positionierung am proximalen oder distalen Ende der Linearführung zu bezeichnen.

Die erfindungsgemäße Wechselvorrichtung ist vorteilhaft geeignet, an einem Roboterarm eines chirurgischen Robotersystem eine Möglichkeit zum automatischen und einfachen Instrumentenwechsel von Schaftinstrumenten zu bieten. Die Wechselvorrichtung ist in der Lage mehrere Schaftinstrumente aufzunehmen, diese über die Schienenführung als geeignete Schnittstelle mit einer Einführ- und Steuerungsmechanik für die Wechselinstrumente in Eingriff zu bringen und die Instrumente anschließend in den Trokar hinein- bzw. wieder herauszufahren, ohne dass eine Person die Instrumente manuell wechseln muss. Das Prinzip lässt sich auf Schaftinstrumente mit verschiedenen Getrieben anwenden.

Gemäß einer weiteren Ausführungsform der erfindungsgemäßen Wechselvorrichtung wird das zumindest eine Eingriffselement mit dem Eingriffsprofil an der Handhabe jedes Wechselinstruments durch zumindest einen Bewegungsaufnehmer bereitgestellt, der zur Ansteuerung des Werkzeugs parallel zu der Instrumentenachse A bewegt werden kann und sich durch eine Öffnung in einer Grundplatte der Handhabe erstreckt, sodass das Eingriffsprofil über die Grundplatte hinausragt. Der Bewegungsaufnehmer kann zur Übertragung einer Bewegung und damit Ansteuerung einer Schwenk- oder Öffnungs- und Schließbewegung des Werkzeugs am distalen Ende des Schafts des Wechselinstruments dienen. Ferner ist an dem Schlitten zumindest ein Bewegungsgeber angeordnet, der in Bezug zu dem Schlitten parallel zu der Längsachse L bewegt werden kann und ein Gegenprofil zum formschlüssigen Eingriff mit dem Eingriffsprofil des Bewegungsaufnehmers bereitstellt. Dabei ragt das Gegenprofil über den Schlitten hinaus. Hierbei wird in der Wechselstellung durch das Gegenprofil des Bewegungsgebers das Führungsprofil des zumindest einem Schienenabschnitts am Schlitten entlang der zumindest einen Kreisbahn bereitgestellt oder ergänzt. D. h., dass entweder der Bewegungsgeber mit dem Gegenprofil in der Wechselstellung als Schienenabschnitt des Schlittens mit dem Führungsprofil dient und somit in der Wechselstellung die Schienenführung des Instrumentenmagazins entlang der zumindest einen Kreisbahn abschnittsweise ergänzt. Oder es sind bevorzugt benachbart zu dem längsbeweglichen Bewegungsgeber Schienenabschnitte an dem Schlitten mit einem dem Führungsprofil entsprechenden Profil ausgebildet, das durch das Gegenprofil des Bewegungsgebers in der Wechselstellung ergänzt wird, sodass die zumindest eine Kreisbahn der Schienenführung des Instrumentenmagazins ununterbrochen auf dem Schlitten in der Wechselstellung verläuft. Hierbei bezieht sich "Wechselstellung" ferner auch auf die Position des längsbeweglichen Bewegungsgebers in Bezug auf den Schlitten.

Für Wechselinstrumente mit mehreren parallel zu der Instrumentenachse A bewegbaren Bewegungsaufnehmern an der Handhabe, deren Eingriffsprofile über die Grundplatte hinausragen, kann die Schienenführung des Instrumentenmagazins und des Schlittens mit entsprechend angepassten Führungsprofilen ausgebildet sein. Da Schlitten zum Eingriff mit den mehreren Bewegungsaufnehmern entsprechend mehrere parallel zur Längsachse L bewegbare Bewegungsgeber aufweist, können deren Gegenprofile in der Wechselstellung die Führungsprofile der Schienenabschnitte am Schlitten entlang der zumindest einen Kreisbahn wie vorbeschrieben bereitstellen oder ergänzen.

Zwischen bzw. neben den Bewegungsgebern, die in der Wechselstellung Schienenabschnitte des Schlittens bereitstellen, kann der Schlitten einen oder mehrere zusätzliche Schienenabschnitte aufweisen, die das Führungsprofil des Instrumentenmagazins ergänzen bzw. es komplettieren. Dies besonders in dem Fall, dass das proximale Ende der Linearführung das Instrumentenmagazin und dessen Schienenführung in zwei Abschnitte, vorzugsweise Hälften, teilt. Diese werden durch den in der Wechselstellung befindlichen Schlitten verbunden, um eine durchgängige Schienenführung mit dem Führungsprofil bereitzustellen, wenn die Bewegungsgeber auch in Wechselstellung ausgerichtet sind, in der das Gegenprofil der Bewegungsgeber das Führungsprofil der Schienenführung ergänzt. So können die Wechselinstrumente entlang der durchgängigen Schienenführung zwischen Passiv- und Wechselstellung bewegt werden, wobei die Bewegungsaufnehmer die Eingriffsprofile bereitstellen, die einerseits zum Gleiten der Wechselinstrumente entlang der Schienenführung und den Schienenabschnitten des Schlittens und andererseits zum Eingriff mit dem Gegenprofil der Bewegungsgeber an dem Schlitten ausgebildet sind. Anders ausgedrückt kann/können der/die Bewegungsgeber am Schlitten in einer bevorzugten Ausführungsform der Wechselvorrichtung einerseits als Schienenabschnitt dienen, dessen Profil in der Wechselstellung das Führungsprofil der Schienenführung ergänzt, und andererseits durch den Eingriff mit dem/den Bewegungsnehmern eines Wechselinstruments als Antriebselement(e) dienen, mit dem/denen das Werkzeug des Wechselinstruments in der Aktivstellung gesteuert wird/werden.

Alle profilierten Bauteilkomponenten, die korrespondierend ineinandergreifend ausgebildet sind, sind entsprechen den Kreisbahnen gekrümmt ausgebildet, so dass ein reibungsloser Eingriff hergestellt und wieder gelöst werden kann. Die durchgängige Schienenführung setzt sich am Schlitten aus den Schienenabschnitten an der Schlittenoberfläche und den entsprechend geformten Bewegungsgebern zusammen. Dabei ist der Eingriff bevorzugt ein formschlüssiger Eingriff, der in Längsrichtung des Wechselinstruments bzw. der Linearführung sperrt, so dass die Instrumente gehalten geführt werden können und damit ein schneller Wechsel möglich ist. Alternativ sind andere Ausführungsformen der Eingriffsprofile möglich, so eine Lücke zwischen den Bewegungsaufnehmern, die dann eine gewisse Breite aufweisen, oder andere dem Fachmann geeignet erscheinende Lösungen.

Der Schlitten weist gemäß noch einer weiteren Ausführungsform der erfindungsgemäßen Wechselvorrichtung eine Positionierautomatik auf, die dazu ausgebildet ist, den zumindest einen Bewegungsgeber bei Anordnung des Schlittens an dem proximalen Ende in der Wechselstellung zu positionieren, sodass das Gegenprofil das Führungsprofil des zumindest einem Schienenabschnitts entlang der zumindest einen Kreisbahn bereitstellt oder ergänzt. Die Positionierautomatik umfasst eine Elektronik- und Aktoreinheit, beispielsweise zumindest einen Motor mit Encoder, der die aktuelle Position des Schlittens und/oder des Bewegungsgebers erkennt und als elektrisches Signal an eine Steuerungseinheit des Motors ausgibt, um den Schlitten und den Bewegungsgeber in die Wechselstellung zu bringen, in der das Gegenprofil des Bewegungsgebers das Führungsprofil entlang der der zumindest einen Kreisbahn ergänzt.

Gemäß noch einer weiteren Ausführungsform der erfindungsgemäßen Wechselvorrichtung wird das zumindest eine Eingriffselement des Wechselinstruments, das das Eingriffsprofil aufweist, durch zwei parallel zur Instrumentenachse A angeordnete Bewegungsaufnehmer zur Ausrichtung einer Taumelscheibe bereitgestellt, die über Lenkdrähte mit einer Schwenkmechanik des Werkzeugs verbunden. Zusätzlich oder alternativ kann das zumindest eine Eingriffselement durch einen in der Instrumentenachse A angeordneten Bewegungsaufnehmer zur Axialbewegung einer Betätigungsstange bereitgestellt werden, wobei die Betätigungsstange mit einer Öffnungs- und Schließmechanik des Werkzeugs verbunden sein kann. Bewegungsgeber und Bewegungsnehmer können ein korrespondierendes Profil aufweisen, so z. B. rechteckige Nuten und Feder oder alternierende Nut-und-Feder-Konstruktionen. Vorteilhaft kann das Getriebe des Wechselinstruments über die Eingriffselemente gesteuert werden.

Jedes Wechselinstrument weist gemäß noch einer weiteren Ausführungsform der erfindungsgemäßen Wechselvorrichtung an der Handhabe zumindest einen Rotationsaufnehmer auf, der zur Ansteuerung des Schafts um eine zur Instrumentenachse A parallele Achse rotiert werden kann und sich durch eine Öffnung in einer Grundplatte der Handhabe erstreckt, sodass der Rotationsaufnehmer über die Grundplatte hinausragt. An dem Schlitten ist zusätzlich zumindest ein Rotationsgeber angeordnet, der um die Längsachse L oder um eine zur Längsachse L parallele Achse rotiert werden kann und zum Eingriff mit dem Rotationsaufnehmer ausgebildet ist. Der Eingriff ist bevorzugt reibschlüssig, so dass der Rotationsaufnehmer durch Reibung an dem Rotationsgeber in Rotation versetzt werden kann und eine einfache Bewegungsübertragung erfolgen kann. Der Rotationsgeber gehört nicht zur Schienenführung, kann diese aber ergänzen.

Gemäß noch einer weiteren Ausführungsform der erfindungsgemäßen Wechselvorrichtung ist der Rotationsaufnehmer als Reibrad ausgebildet und weist ein Elastomermaterial auf. Das Elastomermaterial ist zumindest in dem Abschnitt ausgebildet, in dem das Reibrad mit dem Rotationsgeber in Kontakt stehen kann. Hierbei sind eine elastische Verformbarkeit des Elastomermaterial und ein Überstand des Reibrads über die Grundplatte derart aufeinander abgestimmt, dass das Reibrad zum Wechsel des Wechselinstruments zwischen der Passivstellung und der Wechselstellung bis auf die Grundplatte komprimiert werden kann. Das Reibrad kann als Zwischenrad Drehbewegungen auf die oberhalb der Grundplatte angeordnete Schaftachse übertragen. Zusätzlich oder alternativ kann das Instrumentenmagazin parallel zu der Schienenführung eine Laufbahn zum Abrollen des Reibrads oder eine freigeschnittene Nut entlang dem durch das Instrumentenmagazin definierten Schwenkbereich aufweisen. Alternativ kann der Rotationsaufnehmer aus- und einfahrbar oder schwenkbar an der Handhabe angeordnet sein, sodass der Rotationsaufnehmer in der Passivstellung in die Handhabe eingefahren bzw. eingeschwenkt vorliegt, d. h. nicht über die Grundplatte hinausragt, und bei Überführung in die Wechselstellung aus der Handhabe ausgefahren bzw. ausgeschwenkt wird, um zum Eingriff mit dem Rotationsgeber über die Grundplatte hinauszuragen.

Die Wechselinstrumente können in der Passivstellung unterschiedliche Anordnungen bilden, die durch die Form des Instrumentenmagazins bzw. den Verlauf der Schienenführung bestimmt werden. Gleichwertige, alternative Anordnungen der Instrumente sind eine Fächeranordnung, eine Revolveranordnung, eine kegelförmige Anordnung sowie eine Mischung aus einer Kegel- und Revolveranordnung.

Gemäß einer bevorzugten Ausführungsform der erfindungsgemäßen Wechselvorrichtung sind die Wechselinstrumente in der Passivstellung fächerförmig angeordnet, wobei die zumindest eine Kreisbahn in einer Ebene mit der Längsachse L verläuft und die Instrumentenachsen der Wechselinstrumente in der Passivstellung und der Wechselstellung sich in einem Mittelpunkt R der Kreisbahn schneiden. Dies ist eine besonders platzsparende Variante, vor allem bei einer begrenzten geringen Anzahl an Wechselinstrumenten.

Nach einer ersten alternativen Ausführungsform können die Wechselinstrumente in der Passivstellung auch revolverartig angeordnet sein, wobei die zumindest eine Kreisbahn in zumindest einer Ebene orthogonal zu der Längsachse L verläuft und die Instrumentenachsen der Wechselinstrumente in der Passivstellung parallel zu der Längsachse L vor-liegen und in der Wechselstellung der Längsachse L entspricht. In noch einer weiteren alternativen Ausführungsform können die Wechselinstrumente kegelförmig in einer Kombination der fächerförmigen und revolverartigen Anordnung angeordnet sein, wobei die zumindest eine Kreisbahn in zumindest einer Ebene verläuft, die die Längsachse L in einem von 90° abweichenden Winkel schneidet und die Instrumentenachsen der Wechselinstrumente in der Passivstellung und der Wechselstellung auf einer Mantelfläche eines gedachten Kegels liegen und durch dessen Spitze verlaufen. Die Fächer- bzw. Kegelform bilden sich durch die teilkreisförmige Magazinform, deren Kreismittelpunkt oder Schwenkzentrum in distaler Richtung benachbart zu einem Trokar liegt, der zur Verbindung mit dem Patienten vorgesehen ist. Solche Anordnungen sind platzsparend und ermöglichen einen konstruktiv einfachen Wechselmechanismus für Schaftinstrumente. Um zu vermeiden, dass ein auf dem Schlitten angeordnetes Instrument, dessen Bewegungsaufnehmer mit den Bewegungsgebern am Schlitten in Eingriff stehen, betätigt wird, solange es sich nicht in einer Aktivstellung in der Wechselvorrichtung befindet und nicht im Trokar vorgeschoben ist, kann ein Kontakt der Bewegungsgeber am Schlitten mit den erforderlichen Antriebsmitteln unterbrochen werden, wodurch auch die distale Bewegung der Instrumentenspitze blockiert bzw. eine Bewegung des Werkzeugs nicht ermöglicht wird.

Bei fächerförmiger Anordnung der Wechselinstrumente in der Passivstellung kann gemäß einer weiteren Ausführungsform der erfindungsgemäßen Wechselvorrichtung der Mittelpunkt R der zumindest einen Kreisbahn auf der Längsachse L distal zu dem distalen Ende der Linearführung liegen. Bei revolverartiger Anordnung der Wechselinstrumente in der Passivstellung gemäß der ersten Alternative kann ein Mittelpunkt der zumindest einen Kreisbahn auf einer Mittelachse parallel zu der Längsachse L liegen. Bei kegelförmiger Anordnung der Wechselinstrumente in der Passivstellung kann gemäß der weiteren Alternative ein Mittelpunkt der zumindest einen Kreisbahn auf einer Mittelachse des gedachten Kegels liegen, dessen Spitze auf der Längsachse L distal zu dem distalen Ende der Linearführung liegen.

Das Führungsprofil der Schienenführung des Instrumentenmagazins definiert gemäß einer weiteren Ausführungsform der erfindungsgemäßen Wechselvorrichtung mehrere Kreisbahnen, die parallel um einen gemeinsamen Mittelpunkt R oder eine gemeinsame Mittelachse verlaufen und durch das Führungsprofil des zumindest einen Schienenabschnitts des Schlittens in der Wechselstellung ergänzt werden, wobei das Führungsprofil eine rechteckige, dreieckige oder wellige Kontur aufweist. Diese Konturen als Höhenprofil ermöglichen einen Formschluss zur Antriebsseite für die Kraftübertragung. Die Höhen und Tiefen der Kontur bilden dabei ebenfalls die bereits angesprochene Kreisbahn ab, so dass das Führungsprofil der Schienenführung insgesamt die Kreisbahn abbilden kann.

Die Wechselinstrumente liegen in der Wechselvorrichtung idealerweise eng und platzsparend nebeneinander in einer Fächer- oder Kegelform, berühren sich aber dabei nicht und sind so beabstandet voneinander, dass sie sich nicht gegenseitig in der Bewegung bei Stellungsänderung von Passiv- zu Wechsel- und Aktivstellung behindern. Gemäß noch einer weiteren Ausführungsform der erfindungsgemäßen Wechselvorrichtung weist die Wechselvorrichtung distal zu dem distalen Ende der Linearführung die Trokarvorrichtung oder einen einfachen Trokar zur Führung des Schafts des Wechselinstruments bei Überführung in die Aktivstellung auf. Die Trokarvorrichtung kann dabei mit oder ohne proximalseitigem Einführtrichter zum Einführen des Schafts des Wechselinstruments in die Trokarvorrichtung aufgerüstet sein. Zusätzlich oder alternativ kann in einer weiteren Ausführungsform der erfindungsgemäßen Wechselvorrichtung vorgesehen sein, dass die Wechselvorrichtung distal zu dem distalen Ende der Linearführung eine Führungsvorrichtung aufweist, die dazu ausgebildet ist, das distale Ende des Schafts des Wechselinstruments in der Passivstellung zu halten, beim Wechsel in die Wechselstellung zu stützen und bei der Überführung in die Aktivstellung zu führen. Dazu stellt die Führungsvorrichtung eine Führung des distalen Schaftendes des Wechselinstruments auf einer zu dem Instrumentenmagazin konzentrischen Kreisbahn mit entsprechend kleinerem Radius bereit. Beispielsweise kann die Führungsvorrichtung eine distale Schienenführung ähnlich der Schienenführung des Instrumentenmagazins aufweisen, wobei jedes Wechselinstrument am distalen Schaftende (oder auch am proximalen Ende des Werkzeugs) ein Führungselement mit einem entsprechenden Eingriffsprofil aufweisen.

Bei der fächerförmigen Anordnung der Wechselinstrumente in der Passivstellung kann gemäß einer noch weiteren Ausführungsform der Wechselvorrichtung der Mittelpunkt R der zumindest einen Kreisbahn auf der Längsachse L zwischen dem distalen Ende der Linearführung und dem Trokar oder auch der Trokarvorrichtung liegen. Bei der kegelförmigen Anordnung der Wechselinstrumente in der Passivstellung gemäß der weitern alternativen Ausführungsform der erfindungsgemäßen Wechselvorrichtung kann die Spitze des gedachten Kegels auf der Längsachse L zwischen dem distalen Ende der Linearführung und der Trokarvorrichtung liegen.

Gemäß noch einer weiteren Ausführungsform der erfindungsgemäßen Wechselvorrichtung wird die Linearführung durch eine Führungsschiene oder durch einen Führungskanal bereitgestellt, der zwischen dem proximalen Ende und dem distalen Ende durch Seitenwände begrenzt ist. Dabei ist zwischen der Linearführung und dem Schlitten eine Gleit-oder Wälzschienenführung ausgebildet. Bei Gleitschienenführung werden für Schlitten und Kanal bzw. deren Beschichtungen formstabile und abriebarme Materialien mit geringen Gleitreibungskoeffizienten ausgewählt, die eine gleitreibungsarme Materialpaarung bereitstellen. Bei Wälzschienenführung kann der Schlitten oder der Kanal entsprechende Räder, Rollen, Wälzkörper aufweisen.

Weitere Ausführungsformen der erfindungsgemäßen Wechselvorrichtung beziehen sich darauf, dass auch das Material oder eine Beschichtung der Schienenführung des Instrumentenmagazins und des Schienenabschnitts des Schlittens einerseits und das Material oder eine Beschichtung der Eingriffselemente an der Handhabe der Wechselinstrumente andererseits eine gleitreibungsarme Materialpaarung bereitstellen. Eine Antriebseinheit, mit der die Wechselinstrumente im Instrumentenmagazin entlang der Schienenführung bewegt werden, kann beispielsweise einen zu der Schienenführung konzentrischen Zahnkranz umfassen, der (als Innenzahnkranz) um die Schwenkachse der Wechselinstrumente an einer proximalen Gehäusewand des Instrumentenmagazins oder einem Rahmenelement oder (als Kronenrad) neben der Schienenführung ausgebildet ist. Jedes Wechselinstrument kann über ein entsprechendes Ritzel an der Handhabe mit dem Zahnkranz in Eingriff stehen. Zum Überführen des Schlittens entlang der Linearführung zwischen der Wechselstellung am proximalen Ende und der Aktivstellung am distalen Ende kann die Überführungseinrichtung ebenfalls eine Antriebseinheit, wie z. B. eine Linearspindel, aufweisen. Steuerung und Energieversorgung der Bewegungs-/Rotationsgeber am Schlitten können verdrahtet bzw. über Kontaktschiene erfolgen, ggf. aber auch drahtlos und mit Akku.

Ein erfindungsgemäßes chirurgisches Robotersystem weist zumindest einen Roboterarm und eine Steuerungseinheit auf und umfasst eine erfindungsgemäße Wechselvorrichtung mit zumindest zwei chirurgischen Wechselinstrumenten.

Vorteilhaft wird ein Robotersystem bereitgestellt, das auf dem Master-Slave-Prinzip basieren kann und das in der Lage ist, chirurgische Instrumente während der Operation automatisiert und einfach zu wechseln, d. h. insbesondere sie aufzunehmen, über die oben beschriebene Schnittstelle am Schlitten mit der Einführ- und Steuerungsmechanik in Eingriff zu bringen und die Instrumente anschließend in den Trokar einzufahren bzw. wieder herauszufahren. Dadurch kann notwendiges Personal zur Durchführung einer Operation reduziert oder für andere Aufgaben verwendet werden. Der Instrumentenwechsel kann von direkt der Steuereinheit bzw. einer weiteren, externen Steuereinheit, wie einer Konsole, initiiert werden.

Ein erfindungsgemäßes Verfahren zum Wechseln von chirurgischen Wechselinstrumenten unter Verwendung einer erfindungsgemäßen Wechselvorrichtung umfasst die Schritte
- Auswählen eines der chirurgischen Wechselinstrumente, die in dem Instrumentenmagazin in der Passivstellung aufgenommen sind, wobei die chirurgischen Wechselinstrumente mit dem zumindest einen Eingriffselement an der Handhabe mit der Schienenführung des Instrumentenmagazins, dessen Führungsprofil die zumindest eine Kreisbahn definiert, die sich zu dem proximalen Ende der Linearführung erstreckt, in Eingriff stehen, bevorzugt in Gleiteingriff stehen,
- Anordnen des Schlittens mit dem zumindest einem Schienenabschnitt am proximalen Ende der Linearführung in der Wechselstellung, dabei Ergänzen der Schienenführung des Instrumentenmagazins entlang der zumindest einen Kreisbahn mit dem zumindest einem Schienenabschnitt,
- Aktivieren der Antriebseinheit zum Bewegen des ausgewählten Wechselinstruments aus der Passivstellung entlang der zumindest einen Kreisbahn und Positionieren des ausgewählten Wechselinstruments in der Wechselstellung auf dem Schlitten, und
- Überführen des Schlittens mit dem ausgewählten Wechselinstrument entlang der Linearführung von der Wechselstellung am proximalen Ende der Linearführung in die Aktivstellung am distalen Ende der Linearführung.

Vorteilhaft treten dabei in einem weiteren Schritt des erfindungsgemäßen Verfahrens in der Wechselstellung die Bewegungsgeber am Schlitten mit den Bewegungsaufnehmern am Wechselinstrument in Eingriff und stellen somit auch die Antriebsverbindung zwischen Antriebsmittel für die Handhabe und Handhabe des Wechselinstrument her. Eine manuelle mechanische oder elektronische Verbindung ist nicht notwendig, sondern geschieht damit automatisch. Die Bewegungsaufnehmer übertragen die Bewegung der Bewegungsgeber zur Ansteuerung des Werkzeugs auf die Mechanik der Handhabe. Das Werkzeug kann ab diesem Punkt oder erst nach Überführung des Wechselinstruments in die Aktivstellung angesteuert werden.

In einem weiteren Schritt des erfindungsgemäßen Verfahrens bedingt ein Umkehren der Verfahrensschritte, dass das in Benutzung befindliche Wechselinstrument aus seiner Aktivstellung am distalen Ende der Linearführung entlang der Linearführung zurück in die Wechselstellung am proximalen Ende der Linearführung geführt wird, bis der Schlitten in seiner Anfangsposition angekommen ist und die Schienenabschnitte bzw. deren Profile mit denen des Instrumentenmagazins übereinstimmen bzw. in Kongruenz kommen. Mit erneutem Aktivieren der Antriebseinheit zum Bewegen des ausgewählten Wechselinstruments kann dieses entlang der zumindest einen Kreisbahn aus der Wechselstellung in die Passivstellung von dem Schlitten in das Instrumentenmagazin bewegt werden. Die Bewegungsaufnehmer verlieren den Eingriff mit den Bewegungsgebern auf dem Schlitten, und kommen in Kontakt mit der Schienenführung des Instrumentenmagazins.

Um das geeignete Wechselinstrument auszuwählen, kann in einem weiteren Schritt des erfindungsgemäßen Verfahrens vorgesehen sein, dass ein erstes Wechselinstrument von einer Seite des Instrumentenmagazins auf den Schlitten und darüber hinaus auf die jeweils andere Seite des Instrumentenmagazins positioniert werden kann, um gerade bei einem fächerförmigen Instrumentenmagazin das gewünschte Wechselinstrument auswählen zu können. Bei kegel- oder revolverförmigem Instrumentenmagazin kann alternativ vorgesehen sein, dass die Wechselinstrumente in eine Richtung "im Kreis" geführt werden, solange, bis das gewünschte Wechselinstrument auf dem Schlitten zu liegen kommt.

Weitere Ausführungsformen der Wechselvorrichtung und des chirurgischen Robotersystems sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine perspektivische Ansicht des erfindungsgemäßen chirurgischen Robotersystems,
- **Fig. 2**: eine perspektivische Ansicht der erfindungsgemäßen Wechselvorrichtung mit zwei Wechselinstrumenten in Passivstellung,
- **Fig. 3**: eine perspektivische Ansicht der erfindungsgemäßen Wechselvorrichtung mit zwei Wechselinstrumenten in Passivstellung und einem Wechselinstrument in einer Wechselstellung,
- **Fig. 4**: eine perspektivische Ansicht der erfindungsgemäßen Wechselvorrichtung mit zwei Wechselinstrumenten in Passivstellung und einem Wechselinstrument in einer Aktivstellung,
- **Fig. 5**: eine Detailansicht eines Instrumentenmagazins der erfindungsgemäßen Wechselvorrichtung,
- **Fig. 6**: eine erste Detailansicht einer Grundplatte eines Wechselinstruments für die erfindungsgemäße Wechselvorrichtung, und
- **Fig. 7**: eine weitere Detailansicht einer Grundplatte eines Wechselinstruments für die erfindungsgemäße Wechselvorrichtung.

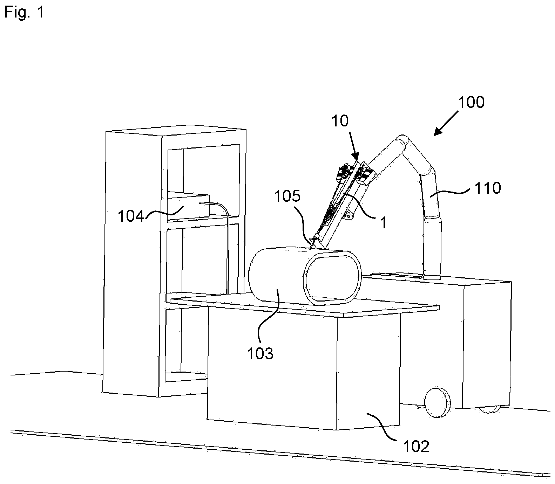

**In** **Fig. 1** weist ein chirurgisches Robotersystem 100 eine Roboterarm 110, eine Steuerungseinheit 104 und eine Wechselvorrichtung 10 zum Wechseln von Wechselinstrumenten 1 auf, wobei letztere Schaftinstrumente mit einem Schaft 3 und einer Handhabe 2 sind, deren distale Enden mit Werkzeugen 4 bestückt sind. Das Wechselinstrument 1 kann über einen Trokar 105, der mit einem auf einem Tisch 102 liegenden Patienten 103 verbunden ist, in letzteren im Rahmen einer minimal-invasiven chirurgischen Behandlung eingesetzt werden. Der Trokar 105 weist einen Einführtrichter 106 auf, der zur Führung des Schafts 3 des Wechselinstruments 1 in den Trokar 105 aufweist.

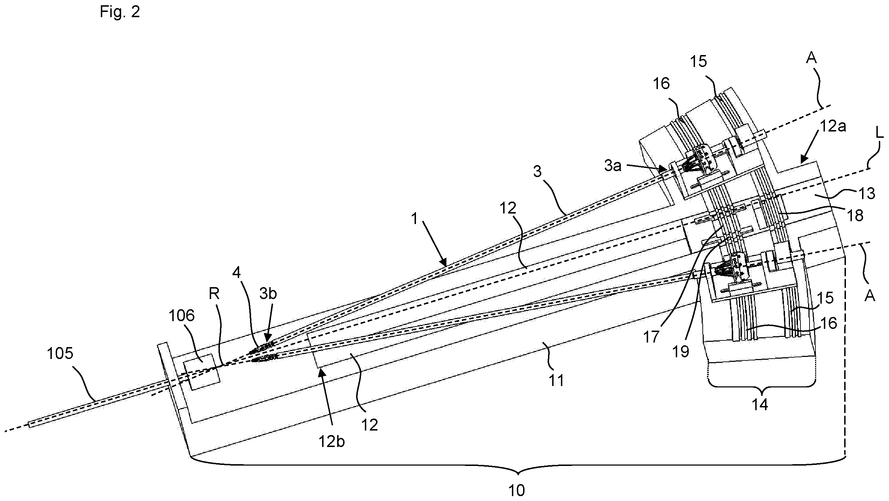

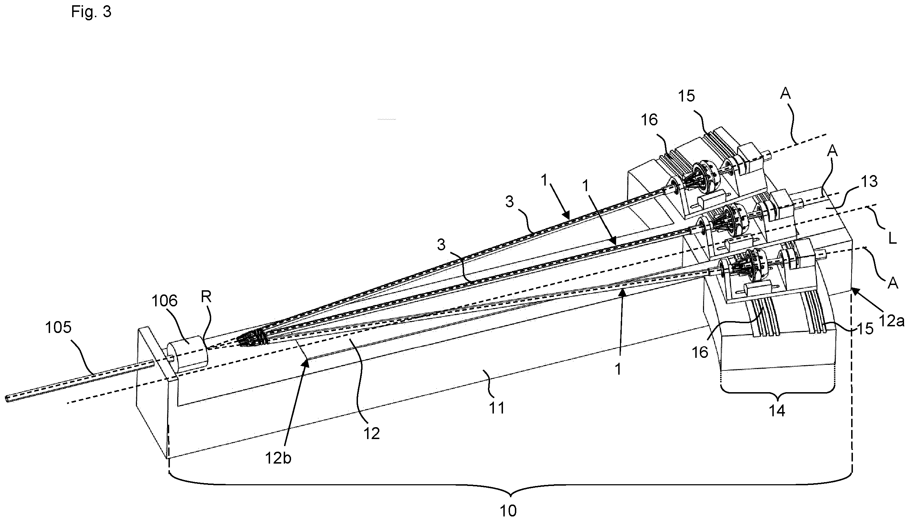

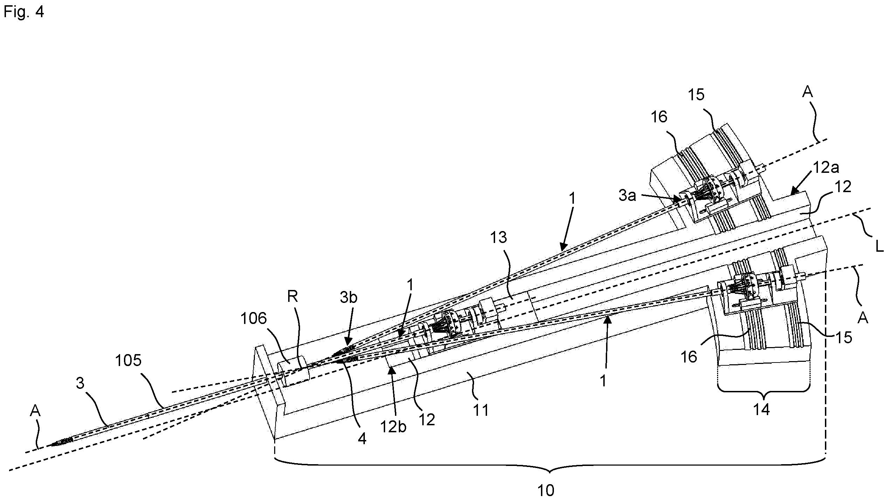

In den **Fig. 2 bis 4** ist die Wechselvorrichtung 10 inklusive zu wechselnder Wechselinstrumente 1 in ihren unterschiedlichen Stellungsmöglichkeiten gezeigt. Ein Wechselinstrument 1 der Wechselvorrichtung 10 kann wahlweise in einer Passivstellung (**Fig. 2**), einer Wechselstellung (**Fig. 3**) und einer Aktivstellung (**Fig. 4**) positioniert werden. Die Wechselvorrichtung 10 weist eine längliche Basis 11 als Grundbauteil auf, an das sich ein Instrumentenmagazin 14 (im Folgenden kurz Magazin 14), in dem zwei Wechselinstrumente 1 in ihrer Passivstellung aufgenommen sind, an einem proximalen Ende 12a der Basis 11 T-förmig anschließt. "Passivstellung" heißt hier, dass die Wechselinstrumente 1 im Magazin 14 gelagert sind und nicht in den Trokar 105 vorgeschoben sind; dies entspräche der "Aktivstellung". In distaler Richtung erstreckt sich die Basis 11 entlang einer Längsachse L. In der Basis ist entlang der Längsachse L eine Linearführung 12 ausgebildet. Die Linearführung 12 ist ein einfacher Führungskanal, der zwischen dem proximalen Ende 12a und dem distalen Ende 12b durch Seitenwände begrenzt ist. In der Linearführung 12 ist ein Schlitten 13 eingesetzt, der entlang der Linearführung 12 verfahren werden kann. Hierfür bilden die Linearführung 12 und der Schlitten 13 eine Gleit- oder Wälzschienenführung. Zum Vorschub des Schlittens 13 sowie zur Bewegung der Wechselinstrumente 1 innerhalb des Magazins 14 ist ein motorischer Antrieb (figurativ nicht dargestellt) vorgesehen.

Die Linearführung 12 bildet zusammen mit dem Schlitten 13 und dem Antrieb eine Überführungseinrichtung zum Überführen eines der Wechselinstrumente 1 zwischen der Wechselstellung am proximalen Ende 12a in die Aktivstellung am distalen Ende 12b der Linearführung 12 (siehe **Fig. 3** **und** **4**).

Das Magazin 14 ist in der in **Fig. 2 bis 5** dargestellten Wechselvorrichtung 10 teilkreisförmig und weist eine Schienenführung aus zwei Schienen 15, 16 mit einem Führungsprofil auf, das in zwei Teilprofilen mehrere Kreisbahnabschnitte definiert, die parallel um einen gemeinsamen Mittelpunkt R verlaufen. Der Schlitten 13 weist zu den zwei Schienen 15, 16, die sich über die gesamte Ausdehnung des Magazins 14 erstrecken, zwei ergänzende Schienenabschnitte 18, 19 auf. Mit dem Schlitten 13 in der Wechselstellung für ein Wechselinstrument 1 in Fig. **2****,** **3** **und** **5** werden so über die gesamte Ausdehnung des Magazins 14 in Querrichtung zu der Längsachse L sich erstreckende Schienen gebildet. Die Schienen 15, 16 und die ergänzenden Schienenabschnitte 18, 19 haben ein gleich ausgebildetes Führungsprofil und ergänzen sich dadurch. Das in den **Fig. 2 bis 5** gezeigte Führungsprofil hat eine rechteckige Kontur.

Die Kreisbahnen der Schienen 15, 16 und der zwei ergänzenden Schienenabschnitte 18, 19 des Schlittens 13 in der Wechselstellung verlaufen in einer Ebene mit der Längsachse L um einen Mittelpunkt R, in dem sich auch die Instrumentenachsen A der Wechselinstrumente 1 in der Passivstellung und der Wechselstellung schneiden. Der Mittelpunkt R liegt auf der Längsachse L distal zu dem distalen Ende 12b der Linearführung 12 und vor dem Trokar 105. Der Radius der Kreisbahnen und damit auch die Länge der Basis 11 und Anordnung des Magazins 14 entlang der Längsachse L orientiert sich dabei an den Abmessungen des Wechselinstruments 1 bzw. der Länge des Schafts 3 des Wechselinstruments 1. Diese teilkreisförmige Ausgestaltung des proximales Abschnitts der Wechselvorrichtung 10 bedingt, dass die im Magazin 14 vorliegenden Wechselinstrumente 1 fächerförmig angeordnet sind. Der Schnittpunkt R, ergo der Radius der Kreisbahnen ist dabei so gewählt, dass die Wechselinstrumente 1 bzw. deren distal angeordnete Werkzeuge 4 sich egal in welcher Stellung oder Position nicht berühren oder sich in ihrer Bewegung behindern. Im Instrumentenmagazin 14 liegen die zwei oder drei Wechselinstrumente 1 zunächst in einer Passivstellung vor. Auf dem Schlitten 13 aufliegend nimmt das dort liegende Wechselinstrument eine Wechselstellung ein. Vorgeschoben in den Trokar 105 ist das auf dem Schlitten aufliegende Wechselinstrument 1 dann in der Aktivstellung.

Jedes in das Magazin 14 eingesetzte Wechselinstrument 1 weist eine Handhabe 2, einen Schaft 3 und das bereits benannte Werkzeug 4 auf. Das Werkzeug 4 ist an einem distalen Ende 3b des Schafts 3 angeordnet und ist mit der Handhabe 2 gekoppelt, die an einem proximalen Ende 3a des Schafts 3 angeordnet ist. Der Schaft definiert eine Instrumentenachse A, die in der Wechselstellung und der Aktivstellung parallel zu einer Längsachse L der Wechselvorrichtung 10 vorliegt. Stutzen 31 kann optional für einen HF-Anschluss des Wechselinstruments 1 genutzt werden.

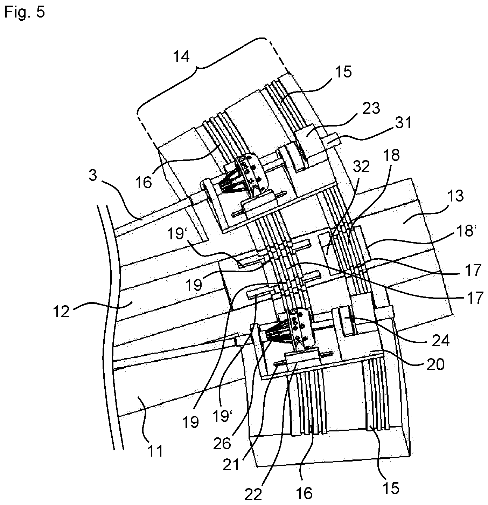

Das Instrumentenmagazin 14 zeigt in Fig. **3** **und** **5** den Schlitten 13 in einer der Wechselstellung entsprechenden Ausgangsstellung am proximalen Ende 12a der Schiene 12/der Wechselvorrichtung 10. Hier wird deutlich, dass der in der Linearführung 12 angeordnete Schlitten 13 Schienenabschnitte 17, 18, 19 an der Schlittenoberfläche aufweist. Die ersten Schienenabschnitte 18, 19 sind jeweils ihrerseits in länglichen Durchgangsöffnungen 18' und einer quadratischen Durchgangsöffnung 19' angeordnet und werden durch Bewegungsgeber 18, 19 bereitgestellt, die wie im Weiteren noch erläutert wird, zur Ansteuerung des Wechselinstruments 1 in der Aktivstellung dienen. Die zweiten Schienenabschnitte 17 sind fest in einer Oberfläche des Schlittens 13 ausgebildet und liegen neben bzw. zwischen den ersten Schienenabschnitten 18, 19, um in der Wechselstellung die Schienen 15, 16 des Magazins 14 zu ergänzen. Dabei ist der Schienenabschnitt 18 in der proximalen Öffnung 18' angeordnet, die die Schiene 15 des Magazins 14 ergänzt. Die Schienenabschnitte 19 sind in den beiden parallel zueinander vorliegenden Öffnungen 19' eingesetzt, um in der Wechselstellung mit der Schiene 16 zu fluchten. Daneben werden die zu dem Magazin 14 vorliegenden "Schienenlücken" auf der Oberfläche des Schlittens 13 durch zweiten Schienenabschnitte 17 neben den Schienenabschnitten 18, 19 aufgefüllt, von denen in **Fig. 5** der Übersichtlichkeit wegen nur zwei bezeichnet sind. Die Schienenabschnitte 18, 19, die durch die Bewegungsgeber 18, 19 bereitgestellt werden, können in Bezug zu dem Schlitten 13 in den Durchgangsöffnungen 18', 19' parallel zu der Längsachse L der Wechselvorrichtung 10 bzw. des Schlittens 13 bewegt werden. In der proximalen Öffnung 18' ist distalseitig benachbart zu dem Schienenabschnitt bzw. Bewegungsgeber 18 ein radförmiger Rotationsgeber 32 angeordnet, der über ein geeignetes Antriebsmittel in Rotation versetzt werden kann. Der Rotationsgeber 32 gehört nicht zu den Führungsprofilen, sondern dient als Antriebsmittel zur Ansteuerung einer Rotationsbewegung des Schafts 3 des Wechselinstruments 1.

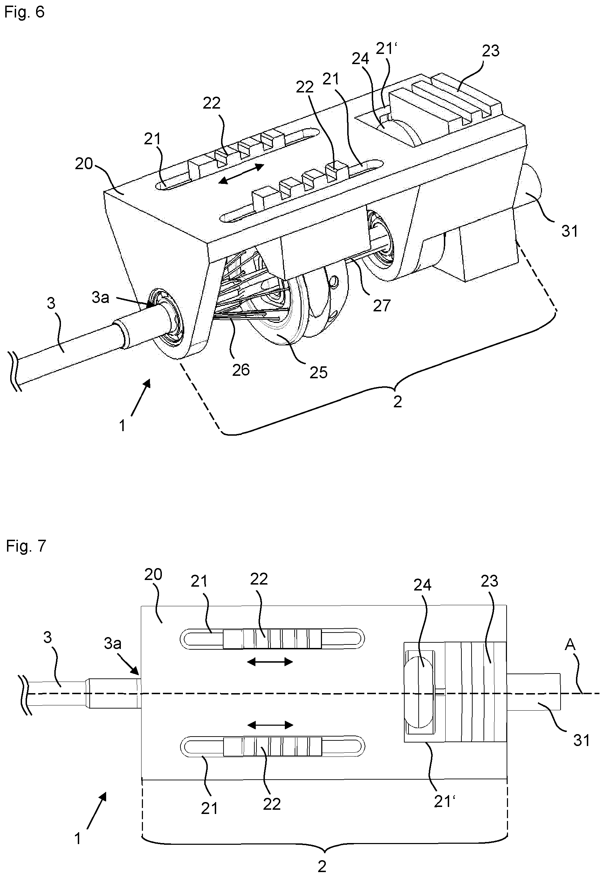

In **Fig. 6 und 7** ist ein proximaler Endabschnitt des Wechselinstruments 1 gezeigt, der sich in proximaler Richtung an das proximale Ende 3a des Schafts 3 anschließt. Dieser proximale Endabschnitt entspricht der Handhabe 2, wobei in **Fig. 6 und 7** die Unterseite der Handhabe 2 gezeigt ist. Die Handhabe 2 jedes Instruments 1 weist eine Grundplatte 20 auf, in die zwei axialparallele längliche Öffnungen 21 und eine mittige proximale Öffnung 21' eingearbeitet sind. In den beiden Öffnungen 21, die links und rechts zur Instrumentenachse A vorliegen, sind jeweils ein Eingriffselement 22, und in die proximale Öffnung 21' ein Eingriffselement 23 mit einem Eingriffsprofil eingesetzt, das über die Grundplatte 20 hinausragt. Das Eingriffsprofil ist komplementär zu dem Führungsprofil der Schienenführung 15, 16 und der Schienenabschnitte 17, 18, 19 ausgebildet, wobei vorliegend das Teilprofil der Eingriffselemente 22 komplementär zu dem Teilprofil der Schiene 16 und der Schienenabschnitte 17, 19 ausgebildet ist, und das Teilprofil des Eingriffselements 23 komplementär zu dem Teilprofil der Schiene 15 und der Schienenabschnitte 17, 18. Damit wird bewirkt, dass jedes Wechselinstrument 1 in der Passivstellung auf der Kreisbahn des Magazins 14 schienengeführt bewegt und in der Wechselstellung auf den Schlitten 13 positioniert werden kann oder auch von dem Schlitten 13 herunterbewegt werden kann. Die Eingriffselemente 22, 23 sind beweglich in den Öffnungen 21, 21' gelagert, und zwar parallel zu der Instrumentenachse A (siehe Pfeile in **Fig. 6 und 7**). Damit dienen sie als Bewegungsaufnehmer 22, 23 der Bewegung, die die Bewegungsgeber 18, 19 des Schlittens 13, also die Schienenabschnitte 18, 19, initiieren. Die Bewegungsaufnehmer 22, 23 übertragen die Bewegung der Bewegungsgeber 18, 19 zur Ansteuerung des Werkzeugs 4 an die Mechanik der Handhabe 2:
Dazu sind die zwei parallel zur Instrumentenachse A angeordneten Bewegungsaufnehmer 22 zur Ausrichtung einer Taumelscheibe 25 vorgesehen. Diese ist über Lenkdrähte 26 mit einer Schwenkmechanik des Werkzeugs 4 verbunden. Über den in der Instrumentenachse A angeordneten Bewegungsaufnehmer 23 wird eine Axialbewegung einer Betätigungsstange 27 ermöglicht, die mit einer Öffnungs- und Schließmechanik des Werkzeugs 4 verbunden ist. Ferner ist benachbart zu dem Bewegungsaufnehmer 23 ein Rotationsaufnehmer 24 angeordnet, der eine Rotationsbewegung des Schafts 3 ansteuert. Der um eine zur Instrumentenachse A parallele Achse drehbare Rotationsaufnehmer 24 erstreckt sich aus der Öffnung 21' in der Grundplatte 20 der Handhabe 2 derart, dass der Rotationsaufnehmer 24 über die Grundplatte 20 hinausragt. Der Schlitten 13 weist dazu korrespondierend den Rotationsgeber 32 auf, der um die Längsachse L oder eine zur Längsachse L parallele Achse drehbewegbar ist und mit dem Rotationsaufnehmer 24 in Eingriff gebracht werden kann. Der Rotationsaufnehmer 24 ist ein Reibrad und weist ein Elastomermaterial auf, sodass er bei Überführung des Wechselinstruments 1 in dem Magazin 14 bis auf die Grundplatte 20 komprimiert werden kann.

### BEZUGSZEICHENLISTE

- 1: Wechselinstrument
- 2: Handhabe
- 3: Schaft
- 3a, 3b: proximales Schaftende, distales Schaftende
- 4: Werkzeug

- 10: Wechselvorrichtung
- 11: Basis
- 12: Linearführung
- 13: Schlitten
- 14: Instrumentenmagazin
- 15: Schienenführung
- 16: Schienenführung
- 17: Schienenabschnitt
- 18: Schienenabschnitt/Bewegungsgeber
- 18': Öffnung
- 19: Schienenabschnitt/Bewegungsgeber
- 19': Öffnung
- 20: Grundplatte
- 21: Öffnung
- 21': Öffnung
- 22: Eingriffselement/Bewegungsaufnehmer
- 23: Eingriffselement/Bewegungsaufnehmer
- 24: Rotationsaufnehmer
- 25: Taumelscheibe
- 26: Lenkdrähte
- 27: Betätigungsstange

- 31: HF-Anschluss
- 32: Rotationsgeber

- 100: chirurgisches Robotersystem
- 102: Tisch
- 103: Patient
- 104: Steuerungseinheit
- 105: Trokar
- 106: Einführtrichter
- 110: Roboterarm

## Patentansprüche

1. Wechselvorrichtung (10) für zumindest zwei chirurgische Wechselinstrumente (1), die in der Wechselvorrichtung (10) wahlweise in einer Aktivstellung, einer Wechselstellung und einer Passivstellung positionierbar sind, wobei die Wechselvorrichtung (10)
- ein Instrumentenmagazin (14) zur Aufnahme der zumindest zwei Wechselinstrumente (1) in der Passivstellung und
- eine Linearführung (12) mit einem proximalen Ende (12a), an dem das Instrumentenmagazin (14) angeordnet ist, und einem distalen Ende (12b), an dem das Wechselinstrument (1) in der Aktivstellung angeordnet ist, und
- eine Antriebseinheit, die dazu ausgebildet ist, wahlweise jedes der Wechselinstrumente (1) an dem proximalen Ende (12a) der Linearführung (12) in der Wechselstellung anzuordnen, und
- eine Überführungseinrichtung zum Überführen des Wechselinstruments (1) entlang der Linearführung (12) zwischen der Wechselstellung am proximalen Ende (12a) und der Aktivstellung am distalen Ende (12b) aufweist,
**dadurch gekennzeichnet, dass**
- jedes Wechselinstrument (1) eine Handhabe (2), einen Schaft (3) und ein Werkzeug (4) aufweist, das an einem distalen Ende (3b) des Schafts (3) angeordnet ist und mit der Handhabe (2) operativ gekoppelt ist, die an einem proximalen Ende (3a) des Schafts (3) angeordnet ist, der eine Instrumentenachse (A) definiert, die in der Wechselstellung und der Aktivstellung parallel zu einer Längsachse (L) der Wechselvorrichtung (10) ist, wobei die Längsachse (L) durch die Linearführung (12) definiert wird, und
- das Instrumentenmagazin (14) eine Schienenführung (15, 16) mit einem Führungsprofil aufweist, das zumindest eine Kreisbahn definiert, die sich zu dem proximalen Ende (12a) der Linearführung (12) erstreckt, und
- die Überführungseinrichtung einen entlang der Linearführung (12) bewegbaren Schlitten (13) mit zumindest einem Schienenabschnitt (17, 18, 19) aufweist, dessen Profil dem Führungsprofil der Schienenführung (15, 16) entspricht und der in der Wechselstellung die Schienenführung (15, 16) des Instrumentenmagazins (14) entlang der zumindest einen Kreisbahn ergänzt,
wobei jedes Wechselinstrument (1) an der Handhabe (2) zumindest ein Eingriffselement (22, 23) mit einem Eingriffsprofil aufweist, das komplementär zu dem Führungsprofil der Schienenführung (15, 16) und des Schienenabschnitts (17, 18, 19) ausgebildet ist, sodass jedes Wechselinstrument (1) in der Passivstellung auf der Kreisbahn des Instrumentenmagazins (14) bewegbar und in der Wechselstellung auf dem Schlitten (13) positionierbar ist.

2. Wechselvorrichtung (10) nach Anspruch 1,
**dadurch gekennzeichnet, dass**
das zumindest eine Eingriffselement (22, 23) des Wechselinstruments (1) mit dem Eingriffsprofil an der Handhabe (2) jedes Wechselinstruments (1) durch zumindest einen Bewegungsaufnehmer (22, 23) bereitgestellt wird, der zur Ansteuerung des Werkzeugs (4) parallel zu der Instrumentenachse (A) bewegbar ist und sich durch eine Öffnung (21) in einer Grundplatte (20) der Handhabe (2) erstreckt, sodass das Eingriffsprofil über die Grundplatte (20) hinausragt, und
an dem Schlitten (13) zumindest ein Bewegungsgeber (18, 19) angeordnet ist, der in Bezug zu dem Schlitten (13) parallel zu der Längsachse (L) bewegbar ist und ein Gegenprofil zum Eingriff mit dem Eingriffsprofil des Bewegungsaufnehmers (22, 23) bereitstellt, wobei das Gegenprofil über den Schlitten (13) hinausragt und in der Wechselstellung entlang der zumindest einen Kreisbahn das Führungsprofil des zumindest einem Schienenabschnitts (17, 18, 19) bereitstellt oder ergänzt.

3. Wechselvorrichtung (10) nach Anspruch 2,
**dadurch gekennzeichnet, dass**
der Schlitten (13) eine Positionierautomatik aufweist, die dazu ausgebildet ist, den zumindest einen Bewegungsgeber (18, 19) bei Anordnung des Schlittens (13) an dem proximalen Ende (12a) in der Wechselstellung zu positionieren, sodass das Gegenprofil das Führungsprofil des zumindest einem Schienenabschnitts (17, 18, 19) entlang der zumindest einen Kreisbahn bereitstellt oder ergänzt.

4. Wechselvorrichtung (10) nach Anspruch 2 oder 3,
**dadurch gekennzeichnet, dass**
das zumindest eine Eingriffselement (22, 23) des Wechselinstruments (1), das das Eingriffsprofil bereitstellt,
- durch zwei parallel zur Instrumentenachse (A) angeordnete Bewegungsaufnehmer (22) zur Ausrichtung einer Taumelscheibe (25) bereitgestellt wird, die über Lenkdrähte (26) mit einer Schwenkmechanik des Werkzeugs (4) verbunden ist, und/oder
- durch einen in der Instrumentenachse (A) angeordneten Bewegungsaufnehmer (23) zur Axialbewegung einer Betätigungsstange bereitgestellt wird, die mit einer Öffnungs- und Schließmechanik des Werkzeugs (4) verbunden ist.

5. Wechselvorrichtung (10) nach zumindest einem der Ansprüche 1 bis 4,
**dadurch gekennzeichnet, dass**
jedes Wechselinstrument (1) an der Handhabe (2) zumindest einen Rotationsaufnehmer (24) aufweist, der zur Ansteuerung des Schafts (3) um eine zu der Instrumentenachse (A) parallele Drehachse bewegbar ist und sich durch eine Öffnung (21') in einer Grundplatte (20) der Handhabe (2) erstreckt, sodass der Rotationsaufnehmer (24) über die Grundplatte (20) hinausragt,
und
an dem Schlitten (13) zumindest ein Rotationsgeber (32) angeordnet ist, der um die Längsachse (L) oder um eine zu der Längsachse (L) parallele Achse drehbewegbar ist und zum Eingriff mit dem Rotationsaufnehmer (24) ausgebildet ist.

6. Wechselvorrichtung (10) nach Anspruch 5,
**dadurch gekennzeichnet, dass**
der Rotationsaufnehmer (24) als Reibrad (24) ausgebildet ist und ein Elastomermaterial aufweist, wobei
- eine elastische Verformbarkeit des Elastomermaterial und ein Überstand des Reibrads (24) über die Grundplatte (20) derart aufeinander abgestimmt sind, dass das Reibrad (24) zum Wechsel des Wechselinstruments (1) zwischen der Passivstellung und der Wechselstellung bis auf die Grundplatte (20) komprimierbar ist, und/oder
- das Instrumentenmagazin (14) parallel zu der Schienenführung (15, 16) eine Laufbahn zum Abrollen des Reibrads (24) aufweist.

7. Wechselvorrichtung (10) nach zumindest einem der Ansprüche 1 bis 6,
**dadurch gekennzeichnet, dass**
die Wechselinstrumente (1) in der Passivstellung
- fächerförmig angeordnet sind, wobei die zumindest eine Kreisbahn in einer Ebene mit der Längsachse (L) verläuft und die Instrumentenachsen (A) der Wechselinstrumente (1) in der Passivstellung und der Wechselstellung sich in einem Mittelpunkt (R) der Kreisbahn schneiden,
oder
- revolverartig angeordnet sind, wobei die zumindest eine Kreisbahn in zumindest einer Ebene orthogonal zu der Längsachse (L) verläuft und die Instrumentenachsen (A) der Wechselinstrumente (1) in der Passivstellung parallel zu der Längsachse (L) vorliegen und in der Wechselstellung der Längsachse (L) entspricht,
oder
- kegelförmig in einer Kombination der fächerförmigen und revolverartigen Anordnung angeordnet sind, wobei die zumindest eine Kreisbahn in zumindest einer Ebene verläuft, die die Längsachse (L) in einem von 90° abweichenden Winkel schneidet und die Instrumentenachsen (A) der Wechselinstrumente (1) in der Passivstellung und der Wechselstellung auf einer Mantelfläche eines gedachten Kegels liegen und durch dessen Spitze verlaufen.

8. Wechselvorrichtung (10) nach Anspruch 7,
**dadurch gekennzeichnet, dass**
- bei fächerförmiger Anordnung der Wechselinstrumente (1) in der Passivstellung der Mittelpunkt (R) der zumindest einen Kreisbahn auf der Längsachse (L) distal zu dem distalen Ende (12b) der Linearführung (12) liegt;
- bei revolverartiger Anordnung der Wechselinstrumente (1) in der Passivstellung ein Mittelpunkt der zumindest einen Kreisbahn auf einer Mittelachse parallel zu der Längsachse (L) liegt;
- bei kegelförmiger Anordnung der Wechselinstrumente (1) in der Passivstellung ein Mittelpunkt der zumindest einen Kreisbahn auf einer Mittelachse des gedachten Kegels liegt, dessen Spitze auf der Längsachse (L) distal zu dem distalen Ende (12b) der Linearführung (12) liegt.

9. Wechselvorrichtung (10) nach zumindest einem der Ansprüche 1 bis 8,
**dadurch gekennzeichnet, dass**
das Führungsprofil der Schienenführung (15, 16) des Instrumentenmagazins (14) mehrere Kreisbahnen definiert, die parallel um einen gemeinsamen Mittelpunkt (R) oder eine gemeinsame Mittelachse verlaufen und durch das Führungsprofil des zumindest einen Schienenabschnitts (18, 19) des Schlittens (13) in der Wechselstellung ergänzt werden,
wobei das Führungsprofil eine rechteckige, dreieckige oder wellige Kontur aufweist.

10. Wechselvorrichtung (10) nach zumindest einem der Ansprüche 1 bis 9,
**dadurch gekennzeichnet, dass**
die Wechselvorrichtung (10) distal zu dem distalen Ende (12b) der Linearführung (12)
- einen Trokar (105) zur Führung des Schafts (3) des Wechselinstruments (1) in der Aktivstellung mit oder ohne proximalseitigem Einführtrichter (106) zum Einführen des Schafts (3) des Wechselinstruments (1) in die Trokarvorrichtung (105) bei Überführung in die Aktivstellung aufweist;
und/oder
- eine Führungsvorrichtung aufweist, die dazu ausgebildet ist, das distalen Ende (3b) des Schafts (3) des Wechselinstruments (1) in der Passivstellung zu halten, beim Wechsel in die Wechselstellung zu stützen und bei der Überführung in die Aktivstellung zu führen.

11. Wechselvorrichtung (10) nach Anspruch 10,
**dadurch gekennzeichnet, dass**
bei der fächerförmigen Anordnung der Wechselinstrumente (1) in der Passivstellung der Mittelpunkt (R) der zumindest einen Kreisbahn auf der Längsachse (L) zwischen dem distalen Ende (12b) der Linearführung (12) und der Trokarvorrichtung (105) liegt;
bei der kegelförmigen Anordnung der Wechselinstrumente (1) in der Passivstellung die Spitze des gedachten Kegels auf der Längsachse (L) zwischen dem distalen Ende (12b) der Linearführung (12) und der Trokarvorrichtung (105) liegt.

12. Wechselvorrichtung (10) nach zumindest einem der Ansprüche 1 bis 11, **dadurch gekennzeichnet, dass**
die Linearführung (12) durch eine Führungsschiene oder durch einen Führungskanal (12) bereitgestellt wird, der zwischen dem proximalen Ende (12a) und dem distalen Ende (12b) durch Seitenwände begrenzt ist,
wobei die Linearführung (12) und der Schlitten (13) eine Gleit- oder Wälzschienenführung bilden.

13. Chirurgisches Robotersystem (100) mit einem Roboterarm (110), einer Steuerungseinheit (104) und einer Wechselvorrichtung (10) nach zumindest einem der Ansprüche 1 bis 12 mit zumindest zwei chirurgischen Wechselinstrumenten (1).

14. Verfahren zum Wechseln von chirurgischen Wechselinstrumenten (1) unter Verwendung einer Wechselvorrichtung (10) nach zumindest einem der Ansprüche 1 bis 13,
**umfassend die Schritte**
- Auswählen eines der chirurgischen Wechselinstrumente (1), die in dem Instrumentenmagazin (14) in der Passivstellung aufgenommen sind, wobei die chirurgischen Wechselinstrumente (1) mit dem zumindest einen Eingriffselement (22, 23) an der Handhabe (2) mit der Schienenführung (15, 16) des Instrumentenmagazins (14), dessen Führungsprofil die zumindest eine Kreisbahn definiert, die sich zu dem proximalen Ende (12a) der Linearführung (12) erstreckt, in Eingriff stehen,
- Anordnen des Schlittens (13) mit dem zumindest einen Schienenabschnitt (18, 19) am proximalen Ende (12a) der Linearführung (12) in der Wechselstellung, dabei Ergänzen der Schienenführung (15, 16) des Instrumentenmagazins (14) entlang der zumindest einen Kreisbahn mit dem zumindest einem Schienenabschnitt (18, 19),
- Aktivieren der Antriebseinheit zum Bewegen des ausgewählten Wechselinstruments (1) aus der Passivstellung entlang der zumindest einen Kreisbahn und Positionieren des ausgewählten Wechselinstruments (1) in der Wechselstellung auf dem Schlitten (13), und
- Überführen des Schlittens (13) mit dem ausgewählten Wechselinstrument (1) entlang der Linearführung (12) von der Wechselstellung am proximalen Ende (12a) der Linearführung (12) in die Aktivstellung am distalen Ende (12b) der Linearführung (12).
